# EUROPEAN PATENT APPLICATION

(11) **EP 2 816 029 A1**
(43) Date of publication of application: **24.12.2014**
(21) Application number: 13172892.5
(22) Date of filing: 19.06.2013
(51) Int. Cl.: C07D 201/08, C07D 223/10

(54) **Process for the preparation of caprolactam**

(71) Applicant: DSM IP Assets B.V., 6411 TE Heerlen (NL)
(72) Inventor: Lefort, Laurent, 6100 AA Echt (NL); Engendahl, Barthel, 6100 AA Echt (NL); De Vries, Johannes Gerardus, 6100 AA Echt (NL)
(74) Representative: Kleiborn, Paul Erik

(57) **Abstract**

The invention provides a process to produce caprolactam comprising contacting hydrogen, dimethyl adipate, and a source of ammonia in the presence of a soluble catalyst system and a protic component. This process allowes the production of caprolactam from renewable sources, e.g. biomass, and can be advantageously done at mild temperatures, saving energy.

## Description

### Field of the invention

The present invention relates to a process to prepare caprolactam.

### Background of the invention

Caprolactam is used as intermediate in the production of polyamide-6. The current annual production is estimated to be 2 billion kg and proceeds via cyclohexanone. Earlier processes involved butadiene. A disadvantage of these processes is that they are based on fossil derived oil and require a lot of energy, and lead to release of greenhouse gases such as CO2 and NOx. Thus, there is a great incentive to replace these processes by other processes leading to less use of energy and in which the amount of greenhouse gases released is substantially less.

One such alternative, sustainable process to produce caprolactam may start from dimethyl adipate. Dimethyl adipate may be obtained by methoxycarbonylation of a mixture of methyl pentenoates (WO2012/131027 and WO2013/131028), which may be obtained in a three-step process from biomass.

In US3,652,549 and JP4800479 is disclosed the production of caprolactam from H₂, NH₃, and DMA using a copper-chromite catalyst; in JP49019250 is disclosed the production of caprolactam from adipic acid or alkyl adipate with NH₃ and Ru on active carbon. A disadvantage of these processes is that they all use heterogeneous catalysts. In addition, very high temperatures are needed, leading to the formation of side products. An additional disadvantage of using copper chromite is that it is highly toxic.

In a first aspect the invention provides a process to produce caprolactam comprising contacting hydrogen, dimethyl adipate, and a source of ammonia in the presence of a soluble catalyst system and a protic component.

The term "a" or "an" as used herein is defined as "at least one" unless specified otherwise. When referring to a noun (e.g. a compound, a cell etc.) in the singular, the plural is understood to be included. Throughout this specification "adipic acid dimethylester" and "dimethyl adipate" are understood to have the same meaning.

The process is preferably carried out under conditions, e.g. of temperature, duration and/or pressure suitable to produce caprolactam.

The catalyst system may comprise a transition metal and a ligand, and optionally a counter-ion. Throughout the specification, the transition metal is also referred to as the catalytic metal. The transition metal may comprise Os, Ru, and/or Fe, preferably the transition metal comprises Ru.

The ligand may be mono-dentate, bi-dentate or tri-dentate, and may comprise the elements P, N, C, and/or O. Preferably the transition metal can be coordinated by one, preferably by more, more preferably by all such elements of the ligand.

The catalyst system may comprise one, two, or more ligands. The ratio ligand / metal can be 1, 1.5, 2, 3, or even more. Alternatively, the ratio can be substoichiometric.

If the ligand is monodentate or bidentate, the catalyst system preferably comprises two, three, or more of such mono- or bidentate ligands, wherein the transition metal may be coordinated by more than one ligand. The catalyst system may also comprise a mixture of two or more different ligands.

The ligand may comprise a tridentate ligand of formula I 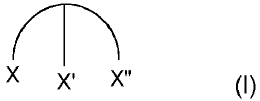 wherein X, X', and X" independently or together represent a phosphine (metal-P bound), an amine (metal-N bound), an alkyl, an aryl group, CO, an eta-6 bound aryl group or an optionally substituted cyclopentadienyl group (metal-C bound), or an ether (metal-O bound).

The ligand may comprise a tridentate ligand, preferably a tridentate ligand of formula II.

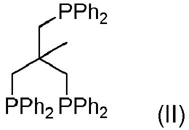

The compound of formula II is also referred to as "TriPhos".

The amount of catalyst system may range between 1/100 and 1/1,000,000 equivalents of the initial dimethyl adipate, preferably between 1/1000 and 1/100,000 equivalents of the initial dimethyl adipate.

In an embodiment, the process may comprise adding a counterion. In the context of the invention, a counterion is any ionic ligand that can bind to a metal. The metal which is part of, or which will form the catalyst system (the "catalytic metal") is typically added in the form of a salt or as a complex. Thus, by adding the catalytic metal to the process, usually a counterion is added concomitantly. However, such catalytic metal counterion is not always the preferred one in view of the chemical reaction. Thus, it may be advantageous to replace the catalytic metal counterion by adding another counterion which is suitable to exchange with the catalytic metal counterion. The counterion can be exchanged by adding an additive suitable to exchange the catalytic counterion. The additive can be in the form of several compounds, as exemplified below.

The counterion can be added in the form of a salt. For example, a chloride ligand (Cl⁻) which may be the counterion of a catalytic metal salt, can be exchanged with tetrafluoroborate (BF4⁻) by adding AgBF4. In this example, BF4- is the added counterion. Alternatively, the counterion can also be added in the form of an acid whose conjugated base may replace the catalytic metal counterion.

The counterion is preferably added to the process separately from the catalytic metal. The added counterion may be a mixture of two or more different counterions.

Examples of suitable counterions which can be added include BArF⁻ (e,.g. as NaBArF), BF₄⁻ (e.g. as AgBF₄), and (AlO(CH₃)X)⁻ (e.g. as methyl aluminium oxane; OAl(CH₃))n, wherein X denotes the counterion initially bound to the metal salt. The additive suitable to exchange the catalytic metals may comprise NaBArF, AgBF4, methyl aluminium oxane (MAO), or LiCl.

The process requires a source of ammonia, which can be ammonia gas or an ammonium salt. The amount of ammonia is at least one equivalent of the initial dimethyl adipate, preferably less than 20 equivalents, more preferably less than 15, even more preferably less than 10 equivalents of the initial dimethyl adipate. If the amount of ammonia is too low, e.g. less than one equivalent of the initial dimethyl adipate, the yield of caprolactam will be suboptimal. If the amount of ammonia is too high, e.g. more than 20 equivalents, the process will be less economical, and/or side products may be formed.

In an embodiment the process comprises a solvent. In the context of the invention, dimethyl adipate is understood not to be a solvent in the process (even though it is liquid at normal reaction temperatures and the reactants may dissolve in it) because it is at least partially consumed in the process. The solvent is preferably an alcohol, more preferably an acidic alcohol, even more preferably it comprises trifluoroethanol.

The protic component may be defined as a compound that can engage into a hydrogen bond relationship with any components of the reaction mixture or that can donate a proton to any components of the reaction mixture. A definition of hydrogen bond relationship can be found in "Arunan, G. R. Desiraju, R. A Klein, J. Sadlej, S. Scheiner, I. Alkorta, D. C. Clary, R. H. Crabtree, J. J. Dannenberg, P. Hobza, H. G. Kjaergaard, A. C. Legon, B. Mennucci, and D. J. Nesbitt (2011). "Defining the hydrogen bond: An Account". Pure Appl. Chem. 83 (8): 1619-1636. doi:10.1351/PAC-REP-10-01-01"

The protic component may be in the form of a solvent. Preferably the protic component comprises a solvent as described above, preferably the protic component comprises an alcohol, preferably the protic component comprises trifluoroethanol.

In an embodiment, the protic component and the solvent are the same. i.e. the solvent may act as the protic component or *vice versa.*

The protic component may also comprise an acid having a pKa of 4 or less, preferably having a pKa of 3 or less, 2 or less, 1 more less, more preferably having a pKa of 0 or less. The acid is preferably an organic acid. Suitable acids include methane sulphonic acid and triflic acid. The acid is usually added to the process in (sub) stoichiometric amounts, for example the amount of the acid can be between 0.01 - 1000, preferably between 0.1 - 100, more between preferably 1-10 mol eq relative to the transition metal.

The temperature range is not critical. For example, a suitable temperature may range between 80 and 200°C.

### EXAMPLES

### Example 1:

Ru(Acac)₃ (9.2mg, 0.023mmol) and TriPhos (16.4mg, 0.026mmol) were placed in a 5mL vials and stirred for 15min in 1mL dry THF under inert atmosphere. The solution was transferred into a 100mL autoclave together with dimethyl adipate (2.15g, 12.34mmol) and 40mL of trifluoroethanol. The autoclave was closed and inertized with N₂ (5 cycles pressurizing the autoclave to 10 bar N₂, releasing the pressure). Liquid NH₃ (2.2g, 0.129mol) was added to the autoclave. The autoclave was pressurized with 50 bar of H₂ and the temperature of the autoclave was increased to 150°C. After 18h, the autoclave was cooled down to room temperature and flushed with N₂. Analysis of the crude mixture by gas chromatography,revealed the presence of caprolactam (10% yield, relative to dimethyl adipate) and adipamide (40% yield, relative to dimethyl adipate) together with other unidentified by-products.

### Example 2:

The procedure was similar to the one described in example 1 except a larger amount of liquid NH₃ was used (4.5g, 0.265mol). Analysis of the crude mixture by gas chromatography, revealed the presence of caprolactam (17% yield, relative to dimethyl adipate) and adipamide (70% yield, relative to dimethyl adipate) together with other unidentified by-products.

## Claims

1. A process to produce caprolactam comprising contacting hydrogen, dimethyl adipate, and a source of ammonia in the presence of a soluble catalyst system and a protic component.

2. Process according to claim 1 wherein the catalyst system comprises a transition metal and a ligand, and optionally a counterion.

3. Process according to claim 1 or 2 wherein the transition metal comprises Os, Ru, and/or Fe, preferably Ru.

4. Process according to claim 2 or 3 wherein said ligand is mono-dentate, bi-dentate or tri-dentate and comprises the elements P, N, C, and/or O.

5. Process according any one of claim 2-4 wherein the ligand comprises a tridentate ligand of formula I 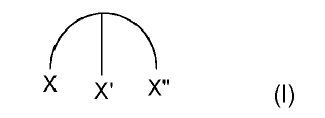 wherein X, X', and X" independently or together represent a phosphine (metal-P bound), an amine (metal-N bound), an alkyl, an aryl group, CO, an eta-6 bound aryl group or an optionally substituted cyclopentadienyl group (metal-C bound), or an ether (metal-O bound).

6. Process according to any one of claim 2 to 5 wherein the ligand comprises a tridentate ligand, preferably a tridentate ligand of formula II

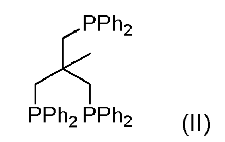

7. Process according to any one of claim 1 to 6 comprising adding an additive suitable to exchange the counterion.

8. Process according to claim 7 wherein the additive comprises NaBArF, AgBF₄, methyl aluminium oxane (MAO), or LiCl.

9. Process according to any one of claim 1 to 8 wherein the amount of ammonia is at least one equivalent, and preferably less than 20 equivalents of the initial dimethyladipate.

10. Process according to any one of claim 1-9 further comprising a solvent.

11. Process according to any one of claim 1 to 10 wherein the protic component is in the form of a solvent.

12. Process according to any one of claim 1-11 whereby the protic component comprises an alcohol, preferably trifluoroethanol.

13. Process according to any one of claim 1 to 12 whereby the protic component comprises an acid having a pKa of 4 or less.

14. Process according to claim 13 whereby the acid comprises an organic acid, preferably triflic acid.

15. Process according to claim 13 or 14 whereby the amount of the acid is between 0.1-100 mol eq relative to the transition metal.
